(19) Europäisches Patentamt
European Patent Office
Office européen des brevets

(11) **EP 4 431 079 A1**

(12) **EUROPÄISCHE PATENTANMELDUNG**

(43) Veröffentlichungstag:
18.09.2024 Patentblatt 2024/38

(21) Anmeldenummer: **23162377.8**

(22) Anmeldetag: **16.03.2023**

(51) Internationale Patentklassifikation (IPC):
*A61K 6/78* (2020.01)        *A61K 6/833* (2020.01)
*A61K 6/836* (2020.01)       *A61K 6/838* (2020.01)
*A61C 13/00* (2006.01)       *C04B 35/01* (2006.01)
*C04B 35/48* (2006.01)       *C04B 35/486* (2006.01)
*C04B 35/488* (2006.01)      *C09C 1/00* (2006.01)

(52) Gemeinsame Patentklassifikation (CPC):
**A61K 6/78; A61C 13/082; A61K 6/833;
A61K 6/836; A61K 6/838; C04B 35/01;
C04B 35/48; C04B 35/486; C04B 35/488;
C09C 1/0009; C09C 1/40;** A61C 13/083;
C01P 2004/61; C01P 2004/62; C01P 2004/64;

(Forts.)

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**KH MA MD TN**

(71) Anmelder: **Ivoclar Vivadent AG
9494 Schaan (LI)**

(72) Erfinder:
• **Serban, Corina
88131 Lindau (DE)**

• **Bolle, Urs
6842 Koblach (AT)**
• **Wildner, Christin
7324 Vilters (CH)**
• **Rothbrust, Frank
6822 Röns (AT)**
• **Krolikowski, Sebastian
8853 Lachen (CH)**
• **Ritzberger, Christian
9472 Grabs (CH)**

(74) Vertreter: **Uexküll & Stolberg
Partnerschaft von
Patent- und Rechtsanwälten mbB
Beselerstraße 4
22607 Hamburg (DE)**

(54) **DENTALMATERIAL MIT PIGMENT**

(57) Die Erfindung betrifft ein Dentalmaterial mit einem Pigment, wobei das Pigment Al, Cr und einen oder mehrere von Y, La und Lanthanoiden enthält. Das Dentalmaterial erlaubt es, die rote Färbung von natürlichen Zähnen und natürlicher Mundschleimhaut nachzuahmen. Gegenstand der Erfindung ist auch die Verwendung des Dentalmaterials, des Pigments und von Ausgangszusammensetzungen zur Herstellung des Pigments bei der Herstellung einer Dentalrestauration. Die Erfindung betrifft ferner ein Verfahren zur Herstellung einer Dentalrestauration sowie ein Verfahren zur Herstellung des Pigments.

EP 4 431 079 A1

(52) Gemeinsame Patentklassifikation (CPC): (Forts.)
C01P 2006/22; C01P 2006/33; C01P 2006/62;
C01P 2006/63; C01P 2006/64

**Beschreibung**

Die Erfindung betrifft ein Dentalmaterial, das ein Pigment enthält.

An Dentalrestaurationen, die z.B. aus Gläsern, Glaskeramiken oder Oxidkeramiken hergestellt sein können, werden hohe Ansprüche in Bezug auf ihre mechanischen Eigenschaften gestellt. Es ist darüber hinaus auch wünschenswert, Dentalrestaurationen ein möglichst natürliches Erscheinungsbild zu verleihen. Dabei gilt es, die Transluzenzeigenschaften von natürlichem Zahnmaterial nachzuahmen und zudem eine möglichst gute farbliche Übereinstimmung der Dentalrestauration mit den verbliebenen natürlichen Zähnen und gegebenenfalls mit dem Farbton der Mundschleimhaut und insbesondere des Zahnfleischs (Gingiva) zu erzielen. Bei der Imitation der Färbung von natürlichen Zähnen und natürlichem Zahnfleisch ist es insbesondere erforderlich, in den Materialien eine Gelb- und eine Rotfärbung zu erzielen.

Es gibt verschiedene Ansätze, um Dentalmaterialien rot einzufärben.

In Gläsern und Glaskeramiken wird eine Rotfärbung insbesondere durch Partikel aus kolloidalem Gold erzielt, wobei dem Glas häufig auch Zinn als Reduktionsmittel zugesetzt wird. Glas, das auf diese Weise rot gefärbt ist, wird auch als Goldrubinglas bezeichnet. Partikel aus kolloidalem Gold können beispielsweise Glasuren oder Schichtmassen beigemischt werden, um eine Rotfärbung von Gläsern oder Schichtmassen zu erzielen, die bei der Herstellung von Dentalrestaurationen verwendet werden. Mit den Partikeln aus kolloidalem Gold können aber nur wenige unterschiedliche rote Farbtöne realisiert werden. Nachteilig ist darüber hinaus, dass die Partikel aus kolloidalem Gold bei den für die Herstellung der Dentalrestauration verwendeten Temperaturen nur sehr eingeschränkt hitzestabil sind. Bei hohen Temperaturen, wie z.B. 1000°C und höher, kommt es deshalb zu einer Abschwächung oder sogar zu einem vollständigen Verlust der roten Farbwirkung. Deshalb können z.B. hochschmelzende Gläser, die für eine Verarbeitung bei einer Temperatur von über 1000°C vorgesehen sind, nicht mit Partikeln aus kolloidalem Gold rot gefärbt werden.

Gläser und Glaskeramiken können auch mit Hilfe von Cadmiumpigmenten, wie insbesondere Cadmiumsulfoselenid Cd(S, Se) rot eingefärbt werden. Wegen ihres hohen toxischen Potenzials dürfen Cadmiumpigmente jedoch nicht bei der Herstellung von Dentalrestaurationen verwendet werden.

Der Erfindung liegt also die Aufgabe zugrunde, Dentalmaterialien mit unterschiedlichen Rotfärbungen bereitzustellen. Die Rotfärbungen sollen beispielsweise die Rotfärbungen der Gingiva oder natürlicher Zähne imitieren können. Darüber hinaus sollen die Rotfärbungen eine hohe Temperaturstabilität aufweisen, damit die Dentalmaterialien beispielsweise den üblicherweise bei der Herstellung von Dentalrestaurationen verwendeten hohen Temperaturen ausgesetzt werden können. Das Dentalmaterial soll auch eine einfache und schnelle Verarbeitung erlauben und zudem kostengünstig sein. Eine gesundheitliche Gefährdung der mit der Herstellung betrauten Personen und der Patienten soll zudem vermieden werden.

Diese Aufgabe wird durch das Dentalmaterial nach den Ansprüchen 1 bis 11 sowie die Verwendung nach den Ansprüchen 12 und 13 gelöst. Die Erfindung ist ebenfalls auf das Verfahren zur Herstellung einer Dentalrestauration nach Anspruch 14 und das Verfahren zur Herstellung des Pigments nach den Ansprüchen 15 bis 17 gerichtet.

Das erfindungsgemäße Dentalmaterial zeichnet sich dadurch aus, dass es ein Pigment enthält, wobei das Pigment Al, Cr, und Z enthält und Z ausgewählt ist aus der Gruppe bestehend aus Y, La, Lanthanoiden und deren Mischungen.

Mit dem erfindungsgemäßen Dentalmaterial können Dentalrestaurationen Farben gegeben werden, die als Farben von natürlichen Zähnen oder von der Mundschleimhaut wahrgenommen werden. Insbesondere erlaubt es das erfindungsgemäße Dentalmaterial, die roten Farbtöne natürlicher Zähne und sogar der natürlichen Mundschleimhaut bei der Herstellung von Dentalrestaurationen zu imitieren.

Es wurde überraschenderweise festgestellt, dass die Pigmente im Dentalmaterial auch bei den hohen Temperaturen stabil sind, die üblicherweise bei der Herstellung von Dentalrestaurationen aus Gläsern, Glaskeramiken oder Feldspatkeramiken verwendet werden. Deshalb ist es besonders vorteilhaft, das erfindungsgemäße Dentalmaterial bei der Herstellung von Dentalrestaurationen zu verwenden.

Es wurde gefunden, dass die hohe Temperaturstabilität auch in dem jeweiligen Gefüge des Dentalmaterials, wie z.B. im Gefüge der Glaskeramik oder der Feldspatkeramik, gegeben ist. Ferner wurde festgestellt, dass das erfindungsgemäße Dentalmaterial mechanische Eigenschaften aufweist wie die entsprechenden herkömmlichen Dentalmaterialien, die das Pigment nicht enthalten. Dies ist überraschend, weil es durch die feine Verteilung von Pigmenten in Dentalmaterialien häufig zu unerwünschten Einflüssen auf das Gefüge kommt. Insbesondere kann es zur Bildung lokaler Eutektika und zu einer Verschlechterung der mechanischen Eigenschaften der Dentalrestauration oder zu unerwünschten Effekten während der Wärmebehandlungen kommen.

Es hat sich zudem gezeigt, dass es die erfindungsgemäßen Dentalmaterialien ermöglichen, in einem kostengünstigen Verfahren eine intensive rote Färbung zu erzielen. Wenn das Dentalmaterial mithilfe des Pigments einfarbig rot eingefärbt ist, werden etwaige nachfolgende Schritte zum Erzeugen einer naturgetreuen Mehrfarbigkeit erleichtert.

Das erfindungsgemäße Dentalmaterial ist auch in Bezug auf das Toxizitätsrisiko und die Biokompatibilität besonders vorteilhaft, da es für die mit der Verarbeitung des Dentalmaterials betrauten Personen und Patienten gesundheitlich unbedenklich ist.

Die Begriffe "Farbe" und "gefärbt" beziehen sich im Sinne der Erfindung auf den Farbwert eines Materials. Farbwerte können durch den L*a*b*-Wert mittels eines Spektrophotometers gemäß DIN 6174 oder durch einen in der Dentalindustrie üblichen Farbschlüssel charakterisiert werden. Die Begriffe "rote Farben" und "rote Farbtöne" beziehen sich auf Farben mit einem positiven a*-Wert im L*a*b*-Farbraum.

Es ist bevorzugt, dass das Pigment Al und Z in einem Molverhältnis von 0,7:1 bis 1:0,7, vorzugsweise 0,8:1 bis 1:0,8 und besonders bevorzugt 0,9:1 bis 1:0,9 enthält.

In einer bevorzugten Ausführungsform enthält das Pigment Z, Al und Cr in einem Molverhältnis entsprechend der Formel $Z_xAl_{2-x-y}Cr_yO_3$, wobei

x 0,8 bis 1,2, vorzugsweise 0,9 bis 1,1, insbesondere 0,95 bis 1,05 und ganz besonders bevorzugt 1,00 beträgt und
y 0,001 bis 0,5, vorzugsweise 0,005 bis 0,25 und insbesondere 0,01 bis 0,1 beträgt.

In einer weiteren bevorzugten Ausführungsform enthält das Pigment zusätzlich Ca.

In einer besonders bevorzugten Ausführungsform hat das Pigment eine Zusammensetzung entsprechend der Formel $Z_xAl_{2-x-y}Cr_yO_3$, wobei x 0,8 bis 1,2, vorzugsweise 0,9 bis 1,1 und insbesondere 0,95 bis 1,05 und ganz besonders bevorzugt 1,00 beträgt und y 0,001 bis 0,5, vorzugsweise 0,005 bis 0,25 und insbesondere 0,01 bis 0,1 beträgt.

In einer weiteren bevorzugten Ausführungsform hat das Pigment eine Zusammensetzung entsprechend der Formel $Z_xAl_{2-x-y}Cr_yO_3$, wobei Z ausgewählt ist aus der Gruppe bestehend aus Yttrium, La und Lanthanoiden, x den Wert 1 aufweist und y 0,001 bis 0,5 beträgt. In diesem Fall hat das Pigment die Formel $ZAl_{1-y}Cr_yO_3$, wobei Z ausgewählt ist aus der Gruppe bestehend aus Yttrium, La und Lanthanoiden und y 0,001 bis 0,5, vorzugsweise 0,005 bis 0,25 und besonders bevorzugt 0,01 bis 0,1 beträgt. Ganz besonders bevorzugt hat das Pigment im Wesentlichen eine Zusammensetzung entsprechend der Formel $YAl_{0,97}Cr_{0,03}O_3$.

Das Molverhältnis von Z, Al und Cr im Pigment sowie die Zusammensetzung des Pigments können z.B. mittels Optischer Emissionsspektroskopie mit induktiv gekoppeltem Plasma (ICP-OES), Massenspektrometrie mit induktiv gekoppeltem Plasma (ICP-MS) oder Atomabsorptionsspektrometrie (AAS) bestimmt werden.

In einer bevorzugten Ausführungsform weist die Hauptkristallphase des Pigments eine Perowskit-Kristallstruktur auf. Der Begriff "Hauptkristallphase" bezeichnet dabei die Kristallphase, die von allen im Pigment vorhandenen Kristallphasen den höchsten Massenanteil hat.

Die Perowskit-Kristallstruktur kann auch verzerrt sein. Der Begriff Perowskit-Kristallstruktur umfasst auch dotierte Perowskit-Kristalle. Das Pigment kann zusätzlich weitere Kristallphasen, wie eine Granatstruktur oder eine monokline Kristallstruktur, aufweisen. Beispielsweise kann, wenn Z Yttrium ist, das Pigment Yttrium-Aluminium-Perowskit (YAP) als Hauptkristallphase und als weitere Kristallphasen Yttrium-Aluminium-Granat (YAG) und/oder eine monokline Yttrium-Aluminium-Kristallphase (YAM) aufweisen.

Die im Pigment enthaltenen Kristallphasen können durch Röntgenbeugungsanalyse (XRD) bestimmt werden. Eine Quantifizierung der Kristallphasen kann insbesondere nach der Rietveld-Methode erfolgen.

Es ist darüber hinaus bevorzugt, dass Z ausgewählt ist aus der Gruppe bestehend aus Y, Er, Pr, Gd, Dy, Eu, Nd, Yb, Ho und Tm. Es hat sich gezeigt, dass sich mit diesen Pigmenten eine besonders intensive Rotfärbung mit hohen a*-Werten erzielen lässt.

In einer bevorzugten Ausführungsform des Dentalmaterials weist das Pigment eine mittlere Partikelgröße $d_{50}$, bestimmt nach ISO 13320, von 0,05 bis 50 $\mu$m, insbesondere 0,1 bis 25 $\mu$m und besonders bevorzugt 0,5 bis 15 $\mu$m auf. Es hat sich gezeigt, dass Partikelgrößen in diesen Bereichen für die mechanischen Eigenschaften des Dentalmaterials besonders vorteilhaft sind. Insbesondere wurde festgestellt, dass die Verwendung kleiner Partikel zur Herstellung von Dentalrestaurationen mit einer besonders hohen Festigkeit vorteilhaft sein kann.

In einer bevorzugten Ausführungsform weist das Pigment eine Farbe auf, deren a*-Wert mindestens 7, insbesondere mindestens 10 beträgt. Es ist besonders bevorzugt, dass zudem der b*-Wert 5 bis 30, insbesondere 10 bis 30, und der L*-Wert 40 bis 65, insbesondere 45 bis 60, beträgt.

In einer weiteren bevorzugten Ausführungsform weist das Pigment eine Farbe auf, deren a*-Wert 7 bis 40, vorzugsweise 10 bis 32, deren b*-Wert 5 bis 30 und deren L*-Wert 40 bis 85 beträgt. Es ist ganz besonders bevorzugt, dass das Pigment eine Farbe aufweist, deren a*-Wert 25 bis 32, deren b*-Wert 15 bis 22 und deren L*-Wert 40 bis 60 beträgt.

Es wurde überraschenderweise festgestellt, dass auch Pigmente, die als Pulver oder in konzentrierter Lösung mit einem braunen Farbton wahrgenommen werden, in Dentalmaterialien Farben bewirken können, die zur Nachahmung der Rotfärbung natürlicher Zähne oder der Mundschleimhaut verwendet werden können.

Es ist bevorzugt, dass das Dentalmaterial in Form eines Pulvers, eines Rohlings oder einer Dentalrestauration vorliegt.

Der Begriff "Dentalmaterial" bezieht sich also unabhängig vom Verfahrensschritt auf jedes Material, das letztendlich zumindest als Teil einer Dentalrestauration dient, die einem Patienten eingesetzt werden kann. In einer bevorzugten Ausführungsform ist das Dentalmaterial ausgewählt aus der Gruppe bestehend aus Dentalmaterial zur Herstellung monolithischer Restaurationen, Verblendmaterial, Material zum Überpressen anderer Materialien, Glasurmaterial, Gerüstmaterial, Haftvermittler und Malfarbe.

Die Dentalrestauration ist vorzugsweise ausgewählt aus der Gruppe bestehend aus Krone, Brücke, Gerüst, Inlay, Onlay, Veneer, Abutment, Teilkrone, Schale, Oberkiefer-Totalprothese, Unterkiefer-Totalprothese, Oberkiefer-Teilprothese und Unterkiefer-Teilprothese.

In einer bevorzugten Ausführungsform enthält das Dentalmaterial ein Glas, eine Glaskeramik, eine Feldspatkeramik oder eine Zusammensetzung zur Herstellung eines Glases, einer Glaskeramik oder einer Feldspatkeramik. Es ist dabei besonders bevorzugt, dass das Glas, die Glaskeramik oder die Zusammensetzung zur Herstellung des Glases oder der Glaskeramik ausgewählt ist aus der Gruppe bestehend aus Gläsern und Glaskeramiken auf Basis von Lithiumsilikat, Leucit, Fluorapatit, Oxyapatit, Quarz, Tiefquarz-Mischkristall, Hochquarz-Mischkristall, Lithiumalumosilikat und deren Mischungen, sowie Zusammensetzungen zu deren Herstellung.

In einer weiteren bevorzugten Ausführungsform ist das Dentalmaterial eine Glaskeramik, vorzugsweise ausgewählt aus der Gruppe bestehend aus Lithiumsilikat-Glaskeramik, Leucit-Glaskeramik, Fluorapatit-Glaskeramik, Oxyapatit-Glaskeramik, Quarz-Glaskeramik, Hochquarz-Mischkristall-Glaskeramik, Tiefquarz-Mischkristall-Glaskeramik und Lithiumalumosilikat-Glaskeramik. Es ist bevorzugt, dass der Anteil aller Kristallphasen in der Glaskeramik insgesamt mindestens 5 Gew.-% beträgt.

Es wurde überraschenderweise festgestellt, dass das Pigment auch zur Einfärbung von Dentalmaterialien, die Glaskeramiken sind, verwendet werden kann. Selbst bei einem hohen Kristallphasenanteil in der Glaskeramik kann die Dentalrestauration mit dem Pigment in dem gewünschten Farbton eingefärbt werden.

Bevorzugt ist ferner, dass das Dentalmaterial ein Glas oder eine Zusammensetzung zu dessen Herstellung ist, wobei das Glas einen dilatometrischen Erweichungspunkt im Bereich von 400 bis 1200°C, insbesondere 420 bis 1150°C und besonders bevorzugt 450 bis 1100°C aufweist. Es ist weiter bevorzugt, dass das Dentalmaterial ein Glas oder eine Zusammensetzung zu dessen Herstellung ist, wobei das Glas einen dilatometrischen Erweichungspunkt im Bereich von 400 bis 600°C, insbesondere 420 bis 580°C und besonders bevorzugt 450 bis 550°C, oder im Bereich von 500 bis 1200°C, insbesondere 600 bis 1150°C und besonders bevorzugt 650 bis 1100°C, aufweist. Im Sinne der vorliegenden Erfindung kann der dilatometrische Erweichungspunkt des Glases nach DIN ISO 6872 bestimmt werden.

In einer bevorzugten Ausführungsform ist das Dentalmaterial ein Glasurmaterial, das bei einer Temperatur von 700°C eine Viskosität von mehr als $10^{2,5}$ Pa·s, bei einer Temperatur von 900°C eine Viskosität von mehr als $10^{2,5}$ Pa·s und/oder bei einer Temperatur von 1100°C eine Viskosität von weniger als $10^9$ Pa.s aufweist.

In einer weiteren bevorzugten Ausführungsform weist das Dentalmaterial eine Farbe mit einem a*-Wert im Bereich von 0 bis 25, insbesondere 1 bis 20 und besonders bevorzugt 2 bis 15 auf.

Es ist zudem bevorzugt, dass das Dentalmaterial eine Farbe mit einem a*-Wert im Bereich von 0 bis 13, vorzugsweise 1 bis 11, besonders bevorzugt 2 bis 8 aufweist. Diese a*-Werte haben sich als besonders vorteilhaft zur Nachahmung der Zahnfarbe erwiesen. Es ist auch bevorzugt, dass das Dentalmaterial eine Farbe mit einem a*-Wert im Bereich von 5 bis 25, vorzugsweise 5 bis 20, besonders bevorzugt 5 bis 15 aufweist. Es wurde festgestellt, dass diese a*-Werte zur Nachahmung der Gingivafarbe besonders geeignet sind. Es ist besonders bevorzugt, dass zusätzlich zu den bevorzugten Bereichen der a*-Werte der b*-Wert 5 bis 35, insbesondere 5 bis 25, und der L*-Wert 65 bis 90, insbesondere 70 bis 90, beträgt.

In einer bevorzugten Ausführungsform enthält das Dentalmaterial das Pigment in einer Menge von 0,005 bis 20 Gew.-%, insbesondere 0,01 bis 16 Gew.-% und besonders bevorzugt 0,05 bis 10 Gew.-%.

Es ist weiter bevorzugt, dass das Dentalmaterial das Pigment in einer Menge von 0,005 bis 5 Gew.-%, insbesondere 0,01 bis 2,5 Gew.-% und besonders bevorzugt 0,05 bis 2 Gew.-% enthält. Es hat sich gezeigt, dass diese Mengen zur Nachahmung der Zahnfarbe besonders geeignet sind. Es ist auch bevorzugt, dass das Dentalmaterial das Pigment in einer Menge von 0,01 bis 20 Gew.-%, insbesondere 0,05 bis 16 Gew.-% und besonders bevorzugt 0,1 bis 10 Gew.-% enthält. Diese Mengen haben sich als besonders geeignet zur Nachahmung der Gingivafarbe erwiesen.

Die Menge des Pigments im Dentalmaterial kann bestimmt werden, indem optische Gefügeanalysen, wie z.B. Elektronenmikroskopie, mit chemischen Analyseverfahren, wie z.B. energiedispersiver Röntgenspektroskopie, kombiniert werden. Dabei wird der Anteil des Pigments im Dentalmaterial zunächst volumetrisch bestimmt und dann unter Berücksichtigung der Dichten des Dentalmaterials und des Pigments in einen Gewichtsanteil umgerechnet. Im Pigment enthaltenen Kristallphasen können durch Röntgenbeugungsanalyse (XRD) bestimmt werden.

Die Erfindung betrifft darüber hinaus auch die Verwendung des vorstehend beschriebenen Dentalmaterials zur Herstellung einer Dentalrestauration sowie die Verwendung des vorstehend beschriebenen Pigments als Dentalmaterial, insbesondere als dentales Färbemittel, oder als Komponente eines Dentalmaterials, insbesondere zur Einfärbung des Dentalmaterials.

In einer bevorzugten Ausführungsform ist die Dentalrestauration ausgewählt aus der Gruppe bestehend aus Krone, Brücke, Gerüst, Inlay, Onlay, Veneer, Abutment, Teilkrone, Schale, Oberkiefer-Totalprothese, Unterkiefer-Totalprothese, Oberkiefer-Teilprothese und Unterkiefer-Teilprothese.

Die erfindungsgemäße Verwendung kann jegliche Verfahrensschritte umfassen, die zur Herstellung einer Dentalrestauration verwendet werden. Beispielsweise kann die Verwendung umfassen, dass dem Dentalmaterial die Form der Dentalrestauration gegeben wird.

In einer bevorzugten Ausführungsform wird das Dentalmaterial, das eine Glaskeramik ist, auf ein Gerüst gepresst. Das Gerüst besteht vorzugsweise aus Metall, Glaskeramik oder Oxidkeramik, insbesondere aus Oxidkeramik auf Basis von $ZrO_2$ oder $Al_2O_3$. Das Pressen umfasst insbesondere das Lost-Wax-Verfahren und es dient vorzugsweise dazu, eine Schicht, die zur Nachahmung der Gingiva in einer roten Farbe eingefärbt ist, auf das Gerüst aufzutragen.

In einer weiteren bevorzugten Ausführungsform wird das Dentalmaterial auf ein Substrat aufgetragen, wobei das Dentalmaterial ein Glas ist, das einen dilatometrischen Erweichungspunkt im Bereich von 400 bis 1200°C, vorzugsweise 420 bis 1150°C und besonders bevorzugt 450 bis 1100°C aufweist. Das Substrat besteht vorzugsweise aus Metall, Glaskeramik oder Oxidkeramik, insbesondere aus Oxidkeramik auf Basis von $ZrO_2$ oder $Al_2O_3$. Es hat sich gezeigt, dass das Pigment auch in diesen hochschmelzenden Gläsern bei den erforderlichen Verarbeitungstemperaturen stabil ist. Deshalb können diese Gläser mit der gewünschten Farbwirkung auf das Substrat aufgebracht werden.

In einer weiteren bevorzugten Ausführungsform wird das Dentalmaterial als Malfarbe verwendet. Die Malfarbe kann mit einem Pinsel auf ein Substrat aufgetragen werden. Vorzugsweise ist die Malfarbe ein Glas mit einem dilatometrischen Erweichungspunkt im Bereich von 400 bis 600°C, insbesondere 420 bis 580°C und besonders bevorzugt 450 bis 550°C, das auf ein Substrat aufgetragen wird, wobei das Substrat insbesondere auf einer Oxidkeramik und/oder Glaskeramik basiert.

In einer weiteren bevorzugten Ausführungsform wird das Dentalmaterial als Glasurmaterial verwendet.

Vorzugsweise ist das Glasurmaterial ein Glas mit einem dilatometrischen Erweichungspunkt im Bereich von 500 bis 1200°C, vorzugsweise 600 bis 1150°C und besonders bevorzugt 650 bis 1100°C. Das Glasurmaterial wird bevorzugt auf ein Substrat aufgetragen, wobei das Substrat insbesondere aus Oxidkeramik oder Metall besteht. Es kann vorteilhaft sein, vor dem Auftragen des Glasurmaterials auf ein Substrat, insbesondere ein Metallsubstrat, zunächst einen Opaker und gegebenenfalls eine Dentin- und Schneidemasse aufzubringen. Vorzugsweise ist das Substrat nicht dichtgesintert, insbesondere ist es ungesintert oder vorgesintert. Es ist bevorzugt, dass das Glasurmaterial im Wesentlichen nicht in das Substrat eindringt. Es wurde festgestellt, dass mit den erfindungsgemäßen Dentalmaterial nicht dichtgesinterte Substrate in einem Schritt beschichtet und rot eingefärbt werden können. Mit den aus dem Stand der Technik bekannten Dentalmaterialien müssen das Beschichten und das Einfärben mit roter Farbe in getrennten Schritten erfolgen.

In einer bevorzugten Ausführungsform der erfindungsgemäßen Verwendung ist das Dentalmaterial ein Glasurmaterial zum Glasieren eines nicht dichtgesinterten Substrats, wobei das Glasurmaterial auf das nicht dichtgesinterte Substrat aufgebracht wird und das Substrat und das Glasurmaterial einer Wärmebehandlung in einem Temperaturbereich unterworfen werden, der sich von einer ersten Temperatur $T_1$ bis zu einer zweiten Temperatur $T_2$ erstreckt, die höher als die erste Temperatur $T_1$ ist, wobei das Glasurmaterial bei der Temperatur $T_1$ eine Viskosität von mehr als $10^{2,5}$ Pa·s, vorzugsweise mehr als $10^{4,0}$ Pa·s, insbesondere mehr als $10^{5,6}$ Pa·s und besonders bevorzugt mehr als $10^{7,0}$ Pa·s, und bei der Temperatur $T_2$ eine Viskosität von weniger als $10^9$ Pa·s, vorzugsweise weniger als $10^7$ Pa·s und insbesondere weniger als $10^{5,6}$ Pa·s aufweist.

Besonders bevorzugt weist das Glasurmaterial bei einer Temperatur von 950°C eine Viskosität von mehr als $10^{2,5}$ Pa-s, insbesondere mehr als $10^{4,0}$ Pa·s, vorzugsweise mehr als $10^{5,6}$ Pa·s und besonders bevorzugt mehr als $10^{7,0}$ Pa·s, bei einer Temperatur von 1300°C eine Viskosität von mehr als $10^{2,5}$ Pa·s und vorzugsweise mehr als $10^4$ Pa·s und bei einer Temperatur von 1450°C eine Viskosität von weniger als $10^9$ Pa·s, vorzugsweise weniger als $10^7$ Pa.s und insbesondere weniger als $10^{5,6}$ Pa.s auf.

In einer weiteren bevorzugten Ausführungsform weist das Glasurmaterial bei einer Temperatur von 700°C eine Viskosität von mehr als $10^{2,5}$ Pa·s, vorzugsweise mehr als $10^{4,0}$ Pa·s, insbesondere mehr als $10^{5,6}$ Pa·s und besonders bevorzugt mehr als $10^{7,0}$ Pa·s, bei einer Temperatur von 900°C eine Viskosität von mehr als $10^{2,5}$ Pa·s und vorzugsweise mehr als $10^4$ Pa·s und bei einer Temperatur von 1100°C eine Viskosität von weniger als $10^9$ Pa·s, vorzugsweise weniger als $10^7$ Pa·s und insbesondere weniger als $10^{5,6}$ Pa·s auf.

Die Bestimmung der Viskosität des Glasurmaterials kann dabei insbesondere anhand einer Viskositäts-Temperatur-Kurve auf Basis der Vogel-Fulcher-Tammann-Gleichung (VFT-Gleichung)

$$log_{10}(\eta) = A + \frac{B}{T - T_0}$$

$\eta$: dynamische Viskosität bei der Temperatur T
A, B, $T_0$: stoffspezifische Konstanten

erfolgen. Diese Gleichung wird ausgehend von mindestens drei und vorzugsweise fünf Wertepaaren aus mittels Dilatometer bzw. Erhitzungsmikroskop experimentell bestimmten charakteristischen Temperaturen und den zugehörigen Viskositätswerten gelöst:

| Bezeichnung und Messmethode | | $\eta$ (Pa·s) |
|---|---|---|
| $T_g$ | (Glasübergangspunkt aus Dilatometer) | 12 |
| $T_d$ | (Erweichungstemperatur aus Dilatometer) | 10 |
| $T_S$ | (Erweichungspunkt aus Erhitzungsmikroskop) | 5,6 |
| $T_{HB}$ | (Halbkugelpunkt aus Erhitzungsmikroskop) | 3,5 |
| $T_F$ | (Fließpunkt aus Erhitzungsmikroskop) | 2,1 |

Die Lösung der Gleichung erfolgt dabei durch ein Näherungsverfahren nach der Methode der kleinsten Quadrate.

In einer weiteren bevorzugten Ausführungsform ist das Pigment in einem Glasurmaterial enthalten, das die Zusammensetzung des in der WO 2018/172544 A1 beschriebenen Glasurmaterials aufweist.

Gegenstand der Erfindung ist auch ein Verfahren zur Herstellung einer Dentalrestauration, bei dem das vorstehend beschriebene Dentalmaterial zu der Dentalrestauration verarbeitet und insbesondere geformt wird oder das vorstehend beschriebene Pigment einem Restaurationsmaterial zugegeben wird und dieses Restaurationsmaterial zu der Dentalrestauration verarbeitet und insbesondere geformt wird.

Alle vorstehend beschriebenen Ausführungsformen des Dentalmaterials und des Pigments sowie der Verfahrensschritte sind auch für das erfindungsgemäße Verfahren zur Herstellung einer Dentalrestauration entsprechend geeignet oder bevorzugt.

Die Erfindung betrifft ferner ein Verfahren zur Herstellung des vorstehend beschriebenen Pigments, bei dem eine Ausgangszusammensetzung kalziniert wird, wobei die Ausgangszusammensetzung $Al(OH)_3$, $Cr_2O_3$, $Z_2O_3$ und gegebenenfalls ein Flussmittel enthält, wobei Z ausgewählt ist aus der Gruppe bestehend aus Y, La und Lanthanoiden, und beim Kalzinieren die Ausgangszusammensetzung von Raumtemperatur auf die Höchsttemperatur mit einer mittleren Aufheizgeschwindigkeit von mindestens 5°C/min erhitzt wird.

In einer bevorzugten Ausführungsform enthält die Ausgangszusammensetzung ein oder mehrere Flussmittel. Geeignete Flussmittel werden auch als Mineralisierungsmittel bezeichnet und sie umfassen Fluoride und Carbonate von Calcium, Natrium und Barium. In einer bevorzugten Ausführungsform enthält die Zusammensetzung als Flussmittel $CaF_2$ oder eine Kombination von $BaCO_2$ und NaF, insbesondere $CaF_2$ oder eine Kombination von $BaCO_3$ und NaF im Molverhältnis 5:1, und besonders bevorzugt $CaF_2$. Es wurde festgestellt, dass unter Verwendung von $CaF_2$, insbesondere in einer Menge von mindestens 3 Gew.-%, besonders intensive rote Farbtöne erzielt werden können.

Es ist zudem bevorzugt, dass Flussmittel, insbesondere $CaF_2$, in einer Menge von 3,0 bis 7,0 Gew.-%, vorzugsweise 4,0 bis 6,0 Gew.-% und besonders bevorzugt 4,5 bis 5,5 Gew.-% in der Zusammensetzung enthalten ist.

Es ist weiter bevorzugt, dass die Ausgangszusammensetzung 0,1 bis 6 Mol-% Cr und insbesondere 1 bis 4 Mol-% Cr, bezogen auf die Menge an Al, enthält.

In einer weiteren bevorzugten Ausführungsform des Verfahrens enthält die Ausgangszusammensetzung eine und vorzugsweise alle der folgenden Komponenten in den angegebenen Mengen

| Komponente | Gew.-% |
|---|---|
| $Z_2O_3$ | 45 bis 65, insbesondere 50 bis 60, |
| $Al(OH)_3$ | 30 bis 45, insbesondere 35 bis 40, |
| $Cr_2O_3$ | 0,7 bis 1,5, insbesondere 0,9 bis 1,3, |
| $CaF_2$ | 3,0 bis 6,0, insbesondere 4,5 bis 5,5, |

wobei Z ausgewählt ist aus Y, La und Lanthanoiden.

In einer bevorzugten Ausführungsform werden die Komponenten der Ausgangszusammensetzung vor dem Kalzinieren zerkleinert und/oder gemischt, wobei das Zerkleinern manuell oder maschinell erfolgen kann und vorzugsweise durch Mahlen erfolgt. Die Komponenten der Ausgangszusammensetzung können auch gleichzeitig oder nacheinander zerkleinert und gemischt werden. Es ist zudem bevorzugt, dass durch das Zerkleinern und Mischen ein im Wesentlichen einheitliches Gemisch der Komponenten der Ausgangszusammensetzung erzeugt wird.

Das Zerkleinern und Mischen kann trocken oder unter Zugabe einer Flüssigkeit durchgeführt werden. Zum Beispiel können die Komponenten der Ausgangszusammensetzung trocken in einem Mörser (z.B. einem Achatmörser) und/oder einer Mörsermühle (z.B. Modell RM200 von Retsch) miteinander gemischt und gemahlen werden.

Die Komponenten der Ausgangszusammensetzung können auch mit Hilfe von $ZrO_2$-Mahlperlen unter Zugabe einer Flüssigkeit, vorzugsweise Wasser und/oder Ethanol, in einem Mischgerät, das die Dual Asymmetrie Centrifuge(DAC)-Technologie verwendet (z.B. SpeedMixer von Hauschild), gemischt und gleichzeitig gemahlen werden. Aus der hergestellten homogenen Suspension können dann mithilfe eines Siebes die $ZrO_2$-Mahlperlen entfernt werden, bevor die Suspension einem Trocknungsschritt unterzogen wird.

Das erfindungsgemäße Verfahren umfasst das Kalzinieren der Ausgangsmaterialien, wodurch Pigmente mit einer roten Färbung erhalten werden. In einer bevorzugten Ausführungsform erfolgt die Kalzinierung bei einer Höchsttemperatur von 1200 bis 1600°C, insbesondere 1250 bis 1550°C und besonders bevorzugt 1300 bis 1500°C.

Vorzugsweise beträgt beim Kalzinieren die mittlere Aufheizgeschwindigkeit, bezogen auf das Erhitzen von Raumtemperatur auf die Höchsttemperatur, 0,1 bis 150°C/min, insbesondere 0,5 bis 100°C/min und besonders bevorzugt 1 bis 75°C/min.

In einer bevorzugten Ausführungsform umfasst das Kalzinieren, dass zunächst auf eine erste Temperatur $T_1$ mit einer Aufheizgeschwindigkeit $R_1$ aufgeheizt wird und dann mit einer zweiten Aufheizgeschwindigkeit $R_2$ bis auf die Höchsttemperatur aufgeheizt wird, wobei $R_1$ größer ist als $R_2$, $T_1$ 400 bis 800°C und vorzugsweise 500 bis 700°C beträgt, $R_1$ 1 bis 100°C/min, vorzugsweise 2 bis 75°C/min und besonders bevorzugt 5 bis 50°C/min beträgt und $R_2$ 1 bis 100°C/min, vorzugsweise 2 bis 75 °C/min und besonders bevorzugt 5 bis 50°C/min beträgt.

Es ist darüber hinaus bevorzugt, dass die Haltezeit der Kalzinierung bei der Höchsttemperatur 0,2 bis 8 h, insbesondere 0,5 bis 6 h beträgt.

Besonders bevorzugt erfolgt die Kalzinierung bei einer Temperatur von 1000 bis 1700°C, insbesondere 1000 bis 1600°C, wobei die Haltezeit 0,2 bis 8 h, insbesondere 0,5 bis 6 h beträgt.

Es ist überraschenderweise festgestellt worden, dass die Aufheizgeschwindigkeit und die Haltezeit den durch das Kalzinieren erzeugten Farbton beeinflussen. Insbesondere führen geringe Aufheizgeschwindigkeiten, wie z.B. eine mittlere Aufheizgeschwindigkeit unter 5°C/min, zu einem Farbton, der als braun wahrgenommen wird.

In einer bevorzugten Ausführungsform werden die Pigmente nach dem Kalzinieren erneut gemahlen und gemischt.

Die Erfindung wird im Folgenden anhand von Ausführungsbeispielen näher erläutert.

**Beispiele**

Beispiele 1 bis 11: Verfahren zur Herstellung des Pigments

Es wurden Pigmente nach dem erfindungsgemäßen Verfahren aus der in Tabelle 1 angegebenen Ausgangszusammensetzung hergestellt.

**Tabelle 1**

| Komponente | Gew.-% |
|---|---|
| $Al(OH)_3$ | 37,7 |
| $Cr_2O_3$ | 1,1 |
| $Z_2O_3$ | 56,2 |
| $CaF_2$ | 5,0 |

Dabei wurde Z in der Komponente $Z_2O_3$ für die Beispiele 1 bis 11 gemäß der Tabelle 2 ausgewählt.

**Tabelle 2**

| Beispiel | Z |
|---|---|
| 1 | Y |
| 2 | Er |
| 3 | Gd |
| 4 | Pr |
| 5 | Dy |

(fortgesetzt)

| Beispiel | Z |
|----------|-----|
| 6 | La |
| 7 | Eu |
| 8 | Nd |
| 9 | Yb |
| 10 | Ho |
| 11 | Tm |

Die Ausgangszusammensetzungen wurden gemischt und mit einem Achatmörser zerkleinert. Dann wurden sie kalziniert, indem sie innerhalb von einer Stunde von Raumtemperatur auf 600°C (Aufheizgeschwindigkeit etwa 9,6°C/min) und dann innerhalb von 2 Stunden auf 1300°C (Aufheizgeschwindigkeit etwa 5,8°C/min) aufgeheizt wurden. Nach einer Haltezeit von einer Stunde bei 1300°C ließ man die Zusammensetzungen frei, d.h. ohne Steuerung der Temperatur, auf Raumtemperatur abkühlen. Es konnte beobachtet werden, dass beim Kalzinieren die zuvor grün gefärbte Zusammensetzung eine Rotfärbung erhielt.

Die L*a*b*-Werte der kalzinierten Pigmente wurden nach DIN 6174 mit einem Spektrophotometer (CM 3700-D, Konica-Minolta) bestimmt. Die dabei ermittelten Werte sind in Tabelle 3 aufgeführt.

**Tabelle 3**

| Beispiel | Z | L* | a* | b* |
|----------|-----|-------|-------|-------|
| 1 | Y | 50,56 | 31,94 | 21,80 |
| 2 | Er | 52,08 | 26,02 | 15,52 |
| 3 | Gd | 46,16 | 20,11 | 13,06 |
| 4 | Pr | 41,16 | 8,72 | 5,98 |
| 5 | Dy | 48,57 | 23,09 | 12,73 |
| 6 | La | 63, 61 | 7,13 | 5, 85 |
| 7 | Eu | 45,87 | 11,05 | 7, 83 |
| 8 | Nd | 44,34 | 12,48 | 7,69 |
| 9 | Yb | 52,56 | 25,42 | 15,83 |
| 10 | Ho | 51,09 | 23,75 | 15,72 |
| 11 | Tm | 56,89 | 24,84 | 15, 63 |

Beispiel 12: Einfluss der Aufheizgeschwindigkeit der Kalzinierung auf die Pigmentfarbe

Der Einfluss der Aufheizgeschwindigkeit beim Kalzinieren auf die Farbe des kalzinierten Pigments wurde untersucht. Dazu wurde eine Ausgangszusammensetzung entsprechend Beispiel 1 hergestellt und kalziniert, indem sie innerhalb von 6 Stunden von Raumtemperatur auf 1300°C (Aufheizgeschwindigkeit etwa 3,6°C/min) aufgeheizt wurde. Nach einer Haltezeit von 6 Stunden bei 1300°C ließ man die Zusammensetzung innerhalb von 2 Stunden auf 900°C und dann frei auf Raumtemperatur abkühlen. Das für dieses Beispiel verwendete Temperaturprofil entsprach also einem üblicherweise verwendeten Verfahren zum Kalzinieren von Pigmenten. Beim Kalzinieren erhielt die Zusammensetzung eine braune Färbung. Die Bestimmung der Farbe des Pigments nach DIN 6174 mit einem Spektrophotometer (CM 3700-D, Konica-Minolta) ergab einen L*-Wert von 40,98, einen a*-Wert von 15,65 und einen b*-Wert von 9, 79.

Beispiele 13 bis 23: Verwendung des Pigments in niedrigsinternden Glasuren

Die Pigmente wurden verwendet, um ein Glas einzufärben, das einen dilatometrischen Erweichungspunkt von 491°C aufweist und als Glasurmaterial vorgesehen ist. Die Zusammensetzung des Glases ist in Tabelle 4 angegeben.

**Tabelle 4**

| Komponente | Gew.-% |
|---|---|
| $SiO_2$ | 65,01 |
| $K_2O$ | 4,55 |
| $Na_2O$ | 7,45 |
| $Li_2O$ | 3,30 |
| CaO | 1,80 |
| SrO | 2,42 |
| $Al_2O_3$ | 5,02 |
| MgO | 0,45 |
| $ZrO_2$ | 0,61 |
| ZnO | 3,50 |
| $B_2O_3$ | 4,02 |
| F | 1,30 |

Das pulverförmige Glas wurde für die Herstellung der Beispiele 13 bis 23 mit jeweils 1 Gew.-% Pigment eingefärbt. Für die Herstellung der Beispiele 13 bis 22 wurden die Lanthan- bzw. Lanthanoid-haltigen Pigmente der Beispiele 1-6 und 8-11 verwendet. Das pulverförmige Glas von Beispiel 23 wurde mit 1 Gew.-% des braunen Pigments von Beispiel 12 eingefärbt. Die für die Herstellung der jeweiligen Beispiele verwendeten Pigmente sind in der Tabelle 5 angegeben.

Die eingefärbten Glaspulver der Beispiele 13 bis 23 wurden bei einer Temperatur von 710°C entsprechend der dentalen Anwendung gebrannt. Dann wurden die L*a*b*-Werte der eingefärbten Gläser mit einem Spektrophotometer (CM 3700-D, Konica Minolta) gemäß DIN 6174 bestimmt. Die gemessenen L*a*b*-Werte sind in der Tabelle 5 angegeben.

**Tabelle 5**

| Beispiel | Z | Pigment aus Bsp. | L* | a* | b* |
|---|---|---|---|---|---|
| 13 | Y | 1 | 45,35 | 35,92 | 23,40 |
| 14 | Er | 2 | 45,91 | 34,20 | 20,49 |
| 15 | Gd | 3 | 42,55 | 29,11 | 17,30 |
| 16 | Pr | 4 | 39,80 | 13,23 | 8,96 |
| 17 | Dy | 5 | 42,42 | 28,12 | 15,00 |
| 18 | La | 6 | 53,71 | 16,58 | 9,82 |
| 19 | Nd | 8 | 41,47 | 19,46 | 10,85 |
| 20 | Yb | 9 | 45,99 | 34,69 | 21,30 |
| 21 | Ho | 10 | 44,27 | 30,98 | 19,34 |
| 22 | Tm | 11 | 49,79 | 34,89 | 21,42 |
| 23 | Y | 12 | 42,87 | 36,61 | 22,57 |

Mit den Pigmenten konnte eine intensive Rotfärbung im Glas erzielt werden. Zwischen den Farben der Gläser 13 und 23 war mit bloßem Auge kein deutlicher Unterschied wahrnehmbar. Dies ist erstaunlich, weil sich die Farbe dieser Y-haltigen Pigmente in Pulverform deutlich unterschied.

Beispiele 24 bis 32: Verwendung des Pigments in einer Fluorapatit-Glaskeramik

Die Pigmente wurden auch verwendet, um eine Fluorapatit-Glaskeramik mit der in Tabelle 6 angegebenen Zusammensetzung einzufärben. Dazu wurde ein entsprechendes Gemenge an Rohstoffen, wie beispielsweise Oxiden, Carbonaten, Phosphaten und Halogeniden bei einer Temperatur von 1500°C für 1 bis 3 h erschmolzen und dann zur Herstellung einer Fritte in Wasser gegossen. Die Fritte wurde getrocknet, gemahlen und zu einem Temperkuchen geformt. Der Temperkuchen wurden für 1 h bei einer Temperatur von 1020°C gehalten, dann in Wasser abgeschreckt, getrocknet und zu einer pulverförmigen Glaskeramik gemahlen.

**Tabelle 6**

| Komponente | Gew.-% |
|---|---|
| $SiO_2$ | 57,68 |
| $K_2O$ | 9,81 |
| $Na_2O$ | 6,37 |
| CaO | 2,72 |
| $Al_2O_3$ | 16,46 |
| $B_2O_3$ | 0,25 |
| $ZrO_2$ | 2,17 |
| $CeO_2$ | 0,51 |
| F | 0,57 |
| ZnO | 1,20 |
| $TiO_2$ | 1,05 |
| $P_2O_5$ | 1,21 |

Die pulverförmige Glaskeramik wurde für die Herstellung der Beispiele 24 bis 33 mit jeweils 1 Gew.-% eingefärbt, wobei die Lanthan- bzw. Lanthanoid-haltigen Pigmente der Beispiele 2-6 und 8-11 verwendet wurden. Die für die Herstellung der jeweiligen Beispiele verwendeten Pigmente sind in der Tabelle 7 angegeben.

**Tabelle 7**

| Beispiel | Z | Pigment aus Bsp. | L* | a* | b* |
|---|---|---|---|---|---|
| 24 | Er | 2 | 52,97 | 30,85 | 18,95 |
| 25 | Gd | 3 | 46,87 | 29,36 | 17,69 |
| 26 | Pr | 4 | 41,87 | 13,57 | 8,39 |
| 27 | Dy | 5 | 49,44 | 27,35 | 14,98 |
| 28 | La | 6 | 57,88 | 14,62 | 8,90 |
| 29 | Nd | 8 | 43,84 | 19,28 | 9,96 |
| 30 | Yb | 9 | 52,99 | 31,40 | 20,56 |
| 31 | Ho | 10 | 50,86 | 28,50 | 19,00 |
| 32 | Tm | 11 | 55,70 | 30,20 | 18,94 |

Mit den Pigmenten konnte eine intensive Rotfärbung in der Glaskeramik erzielt werden.

Beispiele 33 bis 36: Hitzestabilität in Fluorapatit-Glaskeramiken

Zur Herstellung der Beispiele 33 bis 36 wurde die in den Beispielen 24 bis 33 eingesetzte pulverförmige Fluorapatit-Glaskeramik verwendet. Die pulverförmige Glaskeramik wurde für die Herstellung der Beispiele 33 und 34 mit jeweils 1 Gew.-% des Pigments von Beispiel 1 eingefärbt. Für die Herstellung der Beispiele 35 und 36 wurde jeweils 1 Gew.-% des zur Herstellung von Beispiel 17 verwendeten Pigments, dessen Farbe als braun wahrgenommen wird, zur pulverförmigen Glaskeramik gegeben.

Die eingefärbten Glaskeramikpulver wurden entsprechend der dentalen Anwendung für 1 min in einem dentalen Ofen gebrannt, wobei für die Beispiel 33 und 35 jeweils eine Temperatur von 1080°C und für die Beispiele 34 und 36 jeweils eine Temperatur von 1170°C verwendet wurde.

Die L*a*b*-Werte der Glaskeramiken wurden mit einem Spektrophotometer (CM 3700-D, Konica Minolta) gemäß DIN 6174 bestimmt. Die verwendeten Brenntemperaturen sowie die gemessenen Farbwerte sind in Tabelle 8 angegeben.

**Tabelle 8**

| Beispiel | Brenntemperatur (°C) | L* | a* | b* |
|---|---|---|---|---|
| 33 | 1080 | 50,56 | 33,19 | 21,78 |
| 34 | 1170 | 50,82 | 32,89 | 20,99 |
| 35 | 1080 | 48,26 | 34,57 | 21,69 |
| 36 | 1170 | 48,21 | 34,24 | 20,88 |

Ein Vergleich der a*-Werte der Beispiele 33 und 34 beziehungsweise 35 und 36 lässt erkennen, dass eine höhere Brenntemperatur nicht mit einer Verringerung des a*-Werts einhergeht. Dies zeigt, dass die Pigmente selbst bei einer Brenntemperatur von 1170°C in der hochbrennenden Glaskeramik eine sehr gute Stabilität aufweisen.

Wie bereits bei der Verwendung in Gläsern für die Beispiele 13 und 23 beschrieben, kann sowohl mit Pigmenten, die in Pulverform eine Rotfärbung aufweisen, als auch mit Pigmenten, die in Pulverform eine Braunfärbung aufweisen, in Glaskeramiken eine intensive Rotfärbung bewirkt werden. Bei einem Vergleich der Glaskeramiken 33 und 35 beziehungsweise 34 und 36 war mit bloßem Auge kein deutlicher Farbunterschied wahrnehmbar.

**Patentansprüche**

1. Dentalmaterial, das ein Pigment enthält, wobei das Pigment Al, Cr und Z enthält und Z ausgewählt ist aus der Gruppe bestehend aus Y, La, Lanthanoiden und deren Mischungen.

2. Dentalmaterial nach Anspruch 1, bei dem das Pigment Al und Z in einem Molverhältnis von 0,7:1 bis 1:0,7, vorzugsweise 0,8:1 bis 1:0,8 und besonders bevorzugt 0,9:1 bis 1:0,9 enthält.

3. Dentalmaterial nach Anspruch 1 oder 2, bei dem das Pigment Z, Al und Cr in einem Molverhältnis entsprechend der Formel $Z_xAl_{2-x-y}Cr_yO_3$ enthält, wobei

   x 0,8 bis 1,2, vorzugsweise 0,9 bis 1,1, insbesondere 0,95 bis 1,05 und ganz besonders bevorzugt 1,00 beträgt und
   y 0,001 bis 0,5, vorzugsweise 0,005 bis 0,25 und insbesondere 0,01 bis 0,1 beträgt.

4. Dentalmaterial nach einem der Ansprüche 1 bis 3, bei dem die Hauptkristallphase des Pigments eine Perowskit-Kristallstruktur aufweist.

5. Dentalmaterial nach einem der Ansprüche 1 bis 4, bei der Z ausgewählt ist aus der Gruppe bestehend aus Y, Er, Pr, Gd, Dy, Eu, Nd, Yb, Ho und Tm.

6. Dentalmaterial nach einem der Ansprüche 1 bis 5, bei dem das Pigment eine mittlere Partikelgröße $d_{50}$ von 0,05 bis 50 $\mu$m, insbesondere 0,1 bis 25 $\mu$m und besonders bevorzugt 0,5 bis 15 $\mu$m aufweist.

7. Dentalmaterial nach einem der Ansprüche 1 bis 6, das in Form eines Pulvers, eines Rohlings oder einer Dentalrestauration vorliegt.

8. Dentalmaterial nach einem der Ansprüche 1 bis 7, das ein Glas, eine Glaskeramik, eine Feldspatkeramik oder eine Zusammensetzung zur Herstellung eines Glases, einer Glaskeramik oder Feldspatkeramik enthält.

9. Dentalmaterial nach Anspruch 8, bei dem das Glas, die Glaskeramik oder die Zusammensetzung zur Herstellung des Glases oder der Glaskeramik ausgewählt ist aus der Gruppe bestehend aus Gläsern und Glaskeramiken auf Basis von Lithiumsilikat, Leucit, Fluorapatit, Oxyapatit, Quarz, Tiefquarz-Mischkristall, Hochquarz-Mischkristall, Lithiumalumosilikat und deren Mischungen, sowie Zusammensetzungen zu deren Herstellung.

10. Dentalmaterial nach einem der Ansprüche 1 bis 9, das eine Farbe mit einem a*-Wert im Bereich von 0 bis 25, vorzugsweise 1 bis 20, besonders bevorzugt 2 bis 15, oder im Bereich von 5 bis 25, vorzugsweise 5 bis 20 und besonders bevorzugt 5 bis 15 aufweist.

11. Dentalmaterial nach einem der Ansprüche 1 bis 10, das das Pigment in einer Menge von 0,005 bis 20 Gew.-%, vorzugsweise 0,01 bis 16 Gew.-% und besonders bevorzugt 0,05 bis 10 Gew.-% enthält.

12. Verwendung des Dentalmaterials gemäß einem der Ansprüche 1 bis 11 zur Herstellung einer Dentalrestauration oder des in einem der Ansprüche 1 bis 6 definierten Pigments als Dentalmaterial, insbesondere als dentales Färbemittel, oder als Komponente eines Dentalmaterials, insbesondere zur Einfärbung des Dentalmaterials.

13. Verwendung des Dentalmaterials nach Anspruch 12, bei der die Dentalrestauration ausgewählt ist aus der Gruppe bestehend aus Krone, Brücke, Gerüst, Inlay, Onlay, Veneer, Abutment, Teilkrone, Schale, Oberkiefer-Totalprothese, Unterkiefer-Totalprothese, Oberkiefer-Teilprothese und Unterkiefer-Teilprothese.

14. Verfahren zur Herstellung einer Dentalrestauration, bei dem das Dentalmaterial gemäß einem der Ansprüche 1 bis 11 zu der Dentalrestauration verarbeitet und insbesondere geformt wird oder das in einem der Ansprüche 1 bis 6 definierte Pigment einem Restaurationsmaterial zugegeben wird und dieses Restaurationsmaterial zu der Dentalrestauration verarbeitet und insbesondere geformt wird.

15. Verfahren zur Herstellung des in einem der Ansprüche 1 bis 6 definierten Pigments, bei dem eine Ausgangszusammensetzung kalziniert wird, wobei

die Ausgangszusammensetzung $Al(OH)_3$, $Cr_2O_3$, $Z_2O_3$ und gegebenenfalls ein Flussmittel enthält, wobei Z ausgewählt ist aus Y, La und Lanthanoiden, und
beim Kalzinieren die Ausgangszusammensetzung von Raumtemperatur auf die Höchsttemperatur mit einer mittleren Aufheizgeschwindigkeit von mindestens 5°C/min erhitzt wird.

16. Verfahren nach Anspruch 15, bei dem die Ausgangszusammensetzung eine und vorzugsweise alle der folgenden Komponenten in den angegebenen Mengen enthält:

| Komponente | Gew.-% |
|---|---|
| $Z_2O_3$ | 45 bis 65, bevorzugt 50 bis 60, |
| $Al(OH)_3$ | 30 bis 45, bevorzugt 35 bis 40, |
| $Cr_2O_3$ | 0,7 bis 1,5, bevorzugt 0,9 bis 1,3, |
| $CaF_2$ | 3,0 bis 6,0, bevorzugt 4,5 bis 5,5, |

wobei Z ausgewählt ist aus Y, La und Lanthanoiden.

17. Verfahren nach Anspruch 15 oder 16, bei dem das Kalzinieren umfasst, dass auf eine erste Temperatur $T_1$ mit einer Aufheizgeschwindigkeit $R_2$ aufgeheizt wird und dann mit einer zweiten Aufheizgeschwindigkeit $R_2$ bis auf die Höchsttemperatur aufgeheizt wird, wobei $R_1$ größer ist als $R_2$, $T_1$ 400 bis 800°C und vorzugsweise 500 bis 700°C beträgt, $R_1$ 1 bis 100 °C/min, vorzugsweise 2 bis 75°C/min und insbesondere 5 bis 50°C/min beträgt und $R_2$ 1 bis 100°C/min, vorzugsweise 2 bis 75°C/min und insbesondere 5 bis 50°C/min beträgt.

Europäisches Patentamt
European Patent Office
Office européen des brevets

# EUROPÄISCHER RECHERCHENBERICHT

**Nummer der Anmeldung**

EP 23 16 2377

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | EP 3 214 057 A1 (KURARAY NORITAKE DENTAL INC [JP]) 6. September 2017 (2017-09-06) * Absätze [0015] – [0068] * ----- | 1–14 | INV. A61K6/78 A61K6/833 A61K6/836 |
| Y | US 2007/062410 A1 (THIEL NORBERT [DE] ET AL) 22. März 2007 (2007-03-22) * Absätze [0004], [0005], [0024] – [0035]; Beispiele 1–3 * ----- | 1–14 | A61K6/838 A61C13/00 C04B35/01 C04B35/48 C04B35/486 |
| Y | CN 101 624 296 B (UNIV NANCHANG) 6. November 2013 (2013-11-06) * Absätze [0004] – [0019]; Abbildungen 1,2 * ----- | 1–14 | C04B35/488 C09C1/00 |
| Y | CN 102 432 341 B (UNIV EAST CHINA SCIENCE & TECH ET AL.) 11. September 2013 (2013-09-11) * Absätze [0007] – [0035]; Abbildung 2 * ----- | 1–14 | |
| Y | IANOS ROBERT ET AL: "Solution combustion synthesis: a straightforward route for the preparation of chromium-doped lanthanum aluminate, LaAl $_{1-x}$ Cr $_x$ O $_3$ , pink red pigments", DYES AND PIGMENTS, Bd. 155, 1. August 2018 (2018-08-01), Seiten 218-224, XP093075789, GB ISSN: 0143-7208, DOI: 10.1016/j.dyepig.2018.03.041 * "2. Experimental work" and "3. Results and discussion"; Abbildung 4 * ----- | 1–14 | **RECHERCHIERTE SACHGEBIETE (IPC)** A61K C09C A61C C04B |

Der vorliegende Recherchenbericht wurde für alle Patentansprüche erstellt

| Recherchenort | Abschlußdatum der Recherche | Prüfer |
|---|---|---|
| Den Haag | 4. September 2023 | Barenbrug-van Druten |

Europäisches
Patentamt

European
Patent Office

Office européen
des brevets

**Nummer der Anmeldung**

**EP 23 16 2377**

---

## GEBÜHRENPFLICHTIGE PATENTANSPRÜCHE

Die vorliegende europäische Patentanmeldung enthielt bei ihrer Einreichung Patentansprüche, für die eine Zahlung fällig war.

☐ Nur ein Teil der Anspruchsgebühren wurde innerhalb der vorgeschriebenen Frist entrichtet. Der vorliegende europäische Recherchenbericht wurde für jene Patentansprüche erstellt, für die keine Zahlung fällig war, sowie für die Patentansprüche, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die Patentansprüche erstellt, für die keine Zahlung fällig war.

---

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

**Siehe Ergänzungsblatt B**

☐ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☒ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

**1-14**

☐ Der vorliegende ergänzende europäische Recherchenbericht wurde für die Teile der Anmeldung erstellt, die sich auf die zuerst in den Patentansprüchen erwähnte Erfindung beziehen (Regel 164 (1) EPÜ).

Europäisches
Patentamt

European
Patent Office

Office européen
des brevets

## MANGELNDE EINHEITLICHKEIT
## DER ERFINDUNG
## ERGÄNZUNGSBLATT B

**Nummer der Anmeldung**

**EP 23 16 2377**

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

1. Ansprüche: 1-14

    Dentalmaterial, enthältend ein Pigment
    ---

2. Ansprüche: 15-17

    Verfahren zur Herstellung des Pigments
    ---

**ANHANG ZUM EUROPÄISCHEN RECHERCHENBERICHT**
**ÜBER DIE EUROPÄISCHE PATENTANMELDUNG NR.**

EP 23 16 2377

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

04-09-2023

| Im Recherchenbericht angeführtes Patentdokument | Datum der Veröffentlichung | Mitglied(er) der Patentfamilie | Datum der Veröffentlichung |
|---|---|---|---|
| EP 3214057 A1 | 06-09-2017 | CN 107148407 A | 08-09-2017 |
| | | EP 3214057 A1 | 06-09-2017 |
| | | EP 3266439 A1 | 10-01-2018 |
| | | JP 6718377 B2 | 08-07-2020 |
| | | JP WO2016068288 A1 | 05-10-2017 |
| | | KR 20170078694 A | 07-07-2017 |
| | | US 2017247295 A1 | 31-08-2017 |
| | | US 2017334786 A1 | 23-11-2017 |
| | | WO 2016068288 A1 | 06-05-2016 |
| US 2007062410 A1 | 22-03-2007 | KEINE | |
| CN 101624296 B | 06-11-2013 | KEINE | |
| CN 102432341 B | 11-09-2013 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2018172544 A1